# EUROPEAN PATENT APPLICATION

(11) **EP 4 115 888 A1**
(43) Date of publication of application: **11.01.2023**
(21) Application number: 20923234.7
(22) Date of filing: 03.03.2020
(51) Int. Cl.: A61K 31/704, A61P 1/02

(54) **MEDICAL USE OF ANEMOSIDE B4 IN TREATING ORAL ULCER**

(71) Applicant: Liu, Qi, Beijing 100102 (CN)
(72) Inventor: Liu, Qi, Beijing 100102 (CN)
(74) Representative: Altmann Stößel Dick Patentanwälte PartG mbB
(86) International application number: PCT/CN2020/077522
(87) International publication number: WO 2021/174406

(57) **Abstract**

The present invention relates to a new medical use of anemoside B4. In particular, the present invention provides the use of anemoside B4 in the preparation of a medicament for the treatment of oral ulcers; specifically, the use of anemoside B4 in the preparation of a medicament for the treatment of recurrent oral ulcers, and the use of anemoside B4 in the preparation of a medicament for the treatment of oral ulcers caused by chemotherapy.

## Description

### Technical field

The present invention belongs to the field of medicine, and specifically relates to a new medical use of anemoside B4.

### Background technology

Oral ulcer is one of the common oral mucosa diseases, and during development of ulcer, a localized tissue defects in the oral mucosa epithelium is found, and connective tissues are exposed. Because the tissue contains nerves and blood vessels, the patients are very painful after ulcer formation. According to the relevant epidemiological investigation, there are 20% people in our country who have oral ulcer at least once. Regardless of age and gender, any group may have oral ulcer. The disease seriously affects people's diet and speech, and reduces the quality of life in actual life. Clinically, recurrent oral ulcers are the most common, accounting for more than 90% of oral ulcers. The ulcers can be cured, but new ulcer develops soon, characterized by recurrent episodes. The course of the disease is generally 7-10 days, and the interval varies from 10 days to several months. The etiology of oral ulcers is more complicated, which is often the manifestation of local and systemic diseases in oral cavity. Some etiologies are clear, while some are still unclear. However, many studies have shown that oral ulcers are associated with the following factors: genetic, immune disorders, trauma, viral or bacterial infections, autoimmunity, gastrointestinal disorders, anemia, endocrine disorders, nutritional deficiencies, and mental stress.

In addition, oral ulcer is one of the common adverse reactions in patients with malignant tumors during chemotherapy. For patients with malignant tumors, after chemotherapy, the incidence rate of oral ulcer is 66%-75% in domestic literature, while 40%-90% in foreign literature (Jingjing Sun, et al.).The current situation of prevention and treatment of oral ulcers caused by chemotherapy in patients with malignant tumors [J]. Journal of Qilu Nursing, 2013, 19(21): 57-58). Oral ulcers seriously affect patients' feeding, causing nutritional deficiency as well as water-electrolyte imbalance, hindering patients from receiving further treatment, and even developing septicemia, leading to death. The chemotherapeutic agents most likely to cause oral ulcers are methotrexate, fluorouracil, etoposide and doxorubicin.

Since the etiology and pathogenesis are not fully understood, there is no particularly effective treatment for this disease, and clinical therapeutics includes systemic and local treatment. Among them, the drugs used for systemic treatment include glucocorticoids, antimetabolites, vitamins and trace elements, antiviral agents, estrogens, etc. There are also many local treatment methods and drugs, whose main effects are to diminish inflammation, relieve pain and promote healing, and the commonly used medicaments include anti-inflammatory drugs, analgesic drugs, local anesthetics and hormones (local blocking). However, neither systemic nor local treatment has significant effect. In addition, for oral ulcers caused by chemotherapy, glucocorticoids are not conducive to ulcer healing, but will contribute to the worse ulcer. Therefore, it is of great significance to find new drugs with definite therapeutic effects.

Anemoside B4 is a lupane-type pentacyclic triterpene saponin, with the structural formula represented by 1.

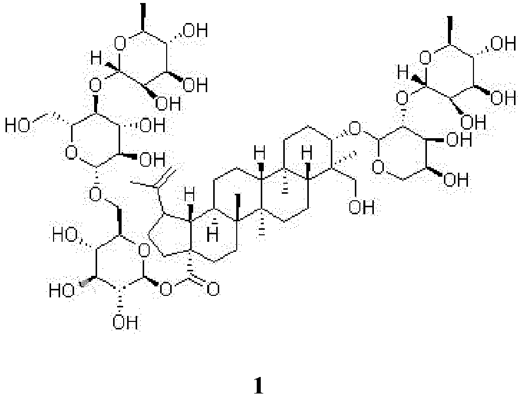

Anemoside B4 has high activity, which has been widely reported in the prior art. For example, anemoside B4 is used as an immunomodulator for the treatment of acute inflammation, including acute kidney injury, acute liver injury, and acute lung injury due to overexpression of inflammatory factors (CN105213410A, published on January 6, 2016). As another example, anemoside B4 was used as an EV71 virus inhibitor for the treatment of Hand, Foot and Mouth Disease (HFMD) (CN105535004A, published on May 4, 2016). For example, on October 18, 2018, Qi Liu filed a Chinese invention patent application entitled "Medical use of anemoside B4 for the treatment of acute gouty arthritis" (application number CN201811214694.3). To date, however, there have been no reports about the therapeutic effect of anemoside B4 on oral ulcers.

### Content of invention

In view of the deficiency in the prior art, the present invention provides a new medical use of anemoside B4, that is, the use of anemoside B4 for the treatment of oral ulcers, especially oral ulcers caused by chemotherapy.

In order to achieve the above invention objective, the following technical solutions are used in the present invention:
anemoside B4 is used in the preparation of a medicament for the treatment of oral ulcers.

As a preferred embodiment, the present invention provides the use of anemoside B4 in the preparation of a medicament for the treatment of recurrent oral ulcers.

As another preferred embodiment, the present invention also provides the use of anemoside B4 in the preparation of a medicament for the treatment of oral ulcers caused by chemotherapy.

Preferably, anemoside B4 is used as the sole active ingredient in the preparation of the medicaments mentioned above.

Alternatively, anemoside B4 is used, together with other active ingredients, in the preparation of the above-mentioned medicaments, in which the other active ingredients are selected from the group consisting of rifampicin, dexamethasone, metronidazole, zinc sulfate, montmorillonite, chlorhexidine, chitosan, sucralfate, polyinosinic-polycytidylic acid (poly I:C), levamisole, vitamin C, B vitamins, granulocyte-macrophage colony stimulating factor (GM-CSF), recombinant human epidermal growth factor (RH EGF), and recombinant human keratinocyte growth factor (rh-KGF).

Preferably, the medicament is selected from one or more of oral and non-oral preparations.

Preferably, the non-oral preparation is selected from the group consisting of injections, rectal preparations, oral mucosa patches, and oral films.

The injection is selected from the group consisting of subcutaneous injections, intramuscular injections, and intravenous injections.

The rectal preparation is selected from the group consisting of rectal suppositories, rectal gels, and rectal perfusion agents.

The pharmaceutically acceptable excipients of the present invention include, but are not limited to (1) diluents, such as starch, powdered sugar, dextrin, lactose, pregelatinized starch, microcrystalline fiber, inorganic calcium salts (such as calcium sulfate, calcium hydrogen phosphate, medicinal calcium carbonate, etc.), mannitol, vegetable oil, polyethylene glycol, cocoa butter, semi- or total-synthetic fatty acid glycerin, gelatin glycerin, etc.; (2) binders, such as distilled water, ethanol, starch slurry, povidone, sodium carboxymethyl cellulose, hydroxypropyl cellulose, methyl cellulose and ethyl cellulose, hydroxypropyl methylcellulose, etc.; (3) disintegrating agents, such as dry starch, sodium carboxymethyl starch (CMS-Na), low-substituted hydroxypropyl cellulose, cross-linked polyvinylpyrrolidone, cross-linked sodium carboxymethyl cellulose, cross-linked povidone, etc.; (4) lubricants, such as magnesium stearate, micronization silica gel, talc, hydrogenated vegetable oil, polyethylene glycol, magnesium lauryl sulfate, etc.; (5) solvents, such as water for injection, ethanol, etc.; (6) preservatives, such as benzoic acid and its salts, sorbic acid and its salts, Nipagin esters, etc.

Preferably, the subject receiving the medicament is a mammal, preferably a human.

When used for human, the dose of anemoside B4 is usually 50 to 300 mg/day for each adult (70 kg); preferably, anemoside B4 is administered once or several times a day for adults (70 kg), with a total dose of 50 to 300 mg for each adult.

### Examples

The present invention will be described below with reference to specific examples. Those skilled in the art will appreciate that these examples are intended to illustrate the present invention only and not to limit the scope of the invention in any way.

The experimental methods in the following examples, unless otherwise specified, are conventional methods. The starting materials, reagents and the like used in the following examples are commercially available, unless otherwise specified.

### Experimental example 1 Effect of anemoside B4 on oral ulcer induced by acetic acid in rats

### 1. The objective of the experiment

The therapeutic effect of anemoside B4 on oral ulcer was evaluated by establishing an acetic acid-induced oral ulcer model in rats and observing the change in food intake and ulcer diameter as well as healing time after the model was established.

### 2. Experimental materials

### 2.1 Experimental animals

SFP SD rats, male, 200-220 g, purchased from Hunan SJA Laboratory Animal Co., Ltd., Certificate No. 43004700060832, License No. SCXK (Xiang) 2016-0002.

### 2.2 Test drugs

Anemoside B4 suppository: self-made, 0.14 g/suppository; based on the content of anemoside B4, there are three specifications: 14 mg/suppository, 7 mg/suppository and 3.5 mg/suppository, batch number: 20190523;
Blank suppository: self-made, prepared in parallel with anemoside B4 suppository, batch number: 20190523-k;
Xilei Powder: Jiangsu 707 Natural Pharmaceutical Co., Ltd., 190102;
Dexamethasone: Henan Runhong Pharmaceutical Co., Ltd., Drug Approval Number H41020330.

The preparation method of anemoside B4 suppository was as follows:
Anemoside B4 is passed through a 100 mesh sieve for later use; and then the matrix (mixed fatty acid glycerides 36# and 38#) is weighed, melted in a water bath at 60 °C, and vigorously stirred, to which was slowly added anemoside B4 under stirring, until anemoside B4 is evenly dispersed in the matrix. The mixture was poured into a plug mold, cooled, and then taken out, to obtain the suppository.

### 2.3 Experimental reagents

Glacial acetic acid (Xilong Science Co., Ltd., batch number 180510), chloral hydrate (Tianjin Damao Chemical Reagent Factory, batch number 20180518), isoflurane (Shenzhen RWD Life Science Co., Ltd., 217180501).

### 2.4 Experimental instruments

Anesthesia machine (RWD, R580), Vernier caliper (Mitutoyo, CD-6"CSX).

### 3. Experimental method

### 3.1 Establishment of rat model of oral ulcer induced by acetic acid

Rats were anesthetized by intraperitoneal injection of 10% chloral hydrate solution. After anesthesia, 3 mm × 3 mm filter paper impregnated with 100% glacial acetic acid was clung to the right buccal mucosa of rats for 45s. After cauterization for 45s, a round or oval white lesion of the buccal mucosa in this area was observed, indicating successful modeling.

### 3.2 Experimental groups

All animals with successful modeling were randomly divided into six groups according to the ulcer area: (1) model group, 14 rats; (2) Xilei powder (15 mg/kg) group, 12 rats; (3) dexamethasone (0.3 mg/kg) group, 13 rats; (4) anemoside B4 high dose (about 60 mg/kg^{∗}3, i.e. 14 mg/suppository, one suppository/time, 3 times/day) group, 14 rats; (5) anemoside B4 medium dose (about 30 mg/kg^{∗}3, i.e. 7 mg/suppository, one suppository/time, 3 times/day) group, 14 rats; (6) anemoside B4 low dose (about 15 mg/kg^{∗}3, i.e. 3.5 mg/suppository, one suppository/time, 3 times/day) group, 14 rats; and 6 rats were included in the normal group.

### 3.3 Administration

24 h after modeling, drug was administrated for 3 successive days, including: local administration in Xilei powder group, once a day; intraperitoneal injection in dexamethasone group, once a day; rectal administration in anemoside B4 group, 3 times a day, and administration of blank suppository in model group, 3 times a day.

### 3.4 Outcome measures

*(1) General observation:* daily food intake per cage was measured from the 2nd day after modeling, and animal weight was measured every 2 days.
*(2) Measurement of ulcer surface diameter:* on the 2^{nd}, 4^{th} and 5^{th} day after modeling, the diameter of ulcer surface was measured with a Vernier caliper and recorded.

### 4. Experimental results

### 4.1 Effect of anemoside B4 suppository on body weight in rats with oral ulcer

The results are shown in Table 1.

**Table 1 Effect of anemoside B4 suppository on body weight of rats with oral ulcer induced by acetic acid B4 (x̅ ± s)**

| Groups | Dose | Body weight (g) | | |
|---|---|---|---|---|
| | | The 2nd day after modeling | The 4th day after modeling | The 6th day after modeling |
| Model group | | 232+10.6 | 230+13.3^{∗} | 238+16.4^{∗} |
| Dexamethasone group | 0.3 mg/kg | 230+8.3 | 216+9.2 | 213+13.7 |
| Xilei powder group | 15 mg/kg | 230+10.8 | 250+9.5^{∗} | 247+14.2^{∗} |
| Anemoside B4 high dose group | 60 mg/kg^{∗}3 | 232+11.3 | 236+19.6^{∗} | 253+9.4^{∗} |
| Anemoside B4 medium dose group | 30 mg/kg^{∗}3 | 230+8.1 | 238+10.7^{∗} | 241+9.7^{∗} |
| Anemoside B4 low dose group | 15 mg/kg^{∗}3 | 229+8.0 | 235+19.9^{∗} | 229+26.9 |

| | | | | |
|---|---|---|---|---|
| Note: ^{∗} indicates p<0.05 compared with dexamethasone group. | | | | |

As shown in Table 1, the body weight of rats in dexamethasone group decreased significantly after administration. Except for dexamethasone group, the body weight of rats in other groups tended to increase after administration compared with the model group. Since the 4^{th} day (3 days after administration), the body weight of rats in anemoside B4 groups had obviously increased compared with dexamethasone group, with significant difference (p<0.05).

### 4.2 Effect of anemoside B4 on food intake in rats with acetic acid-induced oral ulcer

The results are shown in Table 2.

**Table 2 Effect of anemoside B4 suppository on food intake in rats with oral ulcer induced by acetic acid (x̅ ± s)**

| Groups | Dose | Food intake (g)/rat | | |
|---|---|---|---|---|
| | | The 3rd day after modeling | The 4th day after modeling | The 5th day after modeling |
| Normal group | | 26.2±2.83^{∗} | 20.6±3.61 | 21.3±1.26 |
| Model group | | 8.2±1.38 | 12.6±3.37 | 18.0±2.21 |
| Dexamethasone group | 0.3 mg/kg | 13.4±3.35 | 8.2±2.39 | 12.1±4.70 |
| Xilei powder group | 15 mg/kg | 13.3±1.67^{∗} | 18.2±2.37 | 14.8±6.98 |
| Anemoside B4 high dose group | 60 mg/kg^{∗}3 | 11.6±0.95^{∗} | 13.6±1.61 | 19.0±2.86 |
| Anemoside B4 medium dose group | 30 mg/kg^{∗}3 | 12.9±1.83^{∗} | 16.4±2.47 | 16.1±0.80 |
| Anemoside B4 low dose group | 15 mg/kg^{∗}3 | 12.9±3.46 | 13.2±0.76 | 12.8±0.05 |

| | | | | |
|---|---|---|---|---|
| Note: ^{∗} indicates p<0.05 compared with model group. | | | | |

As shown in Table 2, after the model was established, the food intake of rats in model group was significantly decreased compared with the normal group, and especially on the 3^{rd} day after modeling, the least food intake was observed in model group and each drug group, indicating that the ulcer was the most severe on the 3^{rd} day, and the food intake of each group was reduced due to pain. Subsequently, as the ulcer healed, the pain decreased and the food intake gradually increased in each group. On the 3^{rd} day after modeling, when the ulcer was the most severe, the food intake of each anemoside B4 group was higher than that of the model group, and the difference between the high and medium dose groups was statistically significant (p<0.05), indicating that the ulcer injury of each anemoside B4 group was mild. The food intake of dexamethasone group increased on the 3^{rd} day (one day after administration) compared with the model group, but then decreased obviously compared with the model group, and was always less than that of anemoside B4 groups.

### 4.3 Effect of anemoside B4 on the diameter of oral ulcers induced by acetic acid in rats

The results are shown in Table 3.

**Table 3 Effect of anemoside B4 suppository on the diameter of oral ulcers induced by acetic acid in rats (x̅ ± s)**

| Groups | Dose | Diameter of oral ulcer (mm) | | |
|---|---|---|---|---|
| | | The 2nd day after modeling | The 4th day after modeling | The 5th day after modeling |
| Normal group | | - | - | - |
| Model group | | 4.67+0.46 | 3.26+0.35 | 3.26+0.19 |
| Dexamethasone group | 0.3mg/kg | 4.64+0.38 | 3.16+0.18 | 2.78+0.26^{∗} |
| Xilei powder group | 15mg/kg | 4.66+0.41 | 3.43+0.45 | 3.07+0.16 |
| Anemoside B4 high dose group | 60mg/kg^{∗}3 | 4.68+0.41 | 3.03+0.24 | 2.70+0.38^{∗} |
| Anemoside B4 medium dose group | 30mg/kg^{∗}3 | 4.68+0.38 | 2.90+0.30^{∗} | 2.59+0.30^{∗} |
| Anemoside B4 low dose group | 15mg/kg^{∗}3 | 4.66+0.55 | 3.08+0.49 | 2.70+0.23^{∗} |

| | | | | |
|---|---|---|---|---|
| Note: ^{∗} indicates p<0.05 compared with model group. | | | | |

The data in Table 3 showed that anemoside B4 suppository could significantly reduce the diameter of acetic acid-induced oral ulcers in rats from the 4^{th} and 5^{th} day after modeling, having statistical difference (p<0.05) compared with the model group. Its effect of promoting ulcer healing was comparable to that of dexamethasone and better than that of Xilei powder.

### 5. Conclusion

Rectal administration of anemoside B4 had a good therapeutic effect on oral ulcer induced by acetic acid in rats, and its effect of promoting ulcer healing is similar to that of dexamethasone, but significantly better than that of Xilei powder. Meanwhile, anemoside B4 is better than dexamethasone in improving the overall conditions of rats with oral ulcer reflected by body weight and food intake.

### Experimental example 2 Therapeutic effect of anemoside B4 on oral ulcer induced by paclitaxel in rats

### 1. The objective of the experiment

Oral ulcer is a common adverse reaction in cancer patients after radiotherapy and chemotherapy. In this experiment, a rat oral ulcer model induced by chemotherapeutic drug paclitaxel was used to evaluate the therapeutic effect of anemoside B4 on oral ulcer by observing the food intake and ulcer healing time of rats after modeling.

### 2. Experimental materials

### 2.1 Experimental animals

SFP SD rats, male, 180-200 g, purchased from Hunan SJA Laboratory Animal Co., Ltd., Certificate No. 43004700060832, License No. SCXK (Xiang) 2016-0002.

### 2.2 Test drugs

Anemoside B4 suppository: self-made, whose preparation method is the same as that of Example 1; each suppository weights 0.15 g and contains 14 mg of anemoside B4, batch number 20190523.

Blank suppositories were prepared in parallel.

Dexamethasone: Henan Runhong Pharmaceutical Co., Ltd., Drug Approval Number H41020330.

### 2.3 Experimental reagents

Paclitaxel injection (Beijing Shuanglu Pharmaceutical Co., Ltd., batch number 20190101), chloral hydrate (Tianjin Damao Chemical Reagent Factory, batch number 20180518), isoflurane (Shenzhen RWD Life Science Co., Ltd., 217180501).

### 2.4 Experimental instruments

Anesthesia machine (RWD, R580), Vernier caliper (Mitutoyo, CD-6"CSX).

### 3. Experimental method

### 3.1 Establishment of rat model of oral ulcer induced by paclitaxel injection

Rats were anesthetized by intraperitoneal injection of 10% chloral hydrate solution. After anesthesia, the oral ulcer model induced by chemotherapeutic drugs was established by injecting 50 µl of 6 mg/ml paclitaxel injection into the right buccal mucosa of rats.

### 3.2 Experimental groups

All animals with successful modeling were randomly divided into model group, (7 rats), anemoside B4 high dose (about 60 mg/kg^{∗}2) group (7 rats), and normal group (6 rats).

### 3.3 Administration

24 h after modeling, in anemoside B4 group, rectal administration was performed, 1 suppository/time, twice a day, for 5 successive days; in dexamethasone group, intraperitoneal administration was performed, once a day, for 5 successive days; and in model group, blank suppository was given.

### 3.4 Outcome measures

*(1) General observation*: On the day of modeling, rats were fasted but free access to water for 12 hours, while the diet was given quantitatively on the second day after modeling, and the food intake of rats in each cage was measured daily from the third day after modeling (the second day after administration).
*(2) Measurement of ulcer surface diameter:* from the 2^{nd} day after modeling, the diameter of ulcer surface was measured with a Vernier caliper and recorded for 7 successive days.

### 4. Experimental results

### 4.1 Effect of anemoside B4 on food intake in rats with paclitaxel-induced oral ulcer

The results are shown in Table 4.

**Table 4 Effect of anemoside B4 on food intake in rats with oral ulcer induced by paclitaxel (x̅ ± s)**

| Groups | | Food intake/rat (g) | | | |
|---|---|---|---|---|---|
| | The 3rd day after modeling | The 4th day after modeling | The 5th day after modeling | The 6th day after modeling | The 7th day after modeling |
| Normal group | 22.90±1.43* | 20.74±1.14* | 23.35±0.28 | 23.22±2.45 | 27.34±0.66 |
| Model group | 10.97±2.43 | 12.59±0.66 | 16.59±0.49 | 20.80±0.88 | 25.49±0.04 |
| Dexamethasone group | 13.78±3.83 | 10.61±2.48 | 13.91±1.92 | 19.84±1.14 | 18.79±0.56 |
| Anemoside B4 group | 17.88±0.38* | 19.13±0.23* | 21.56±0.88 | 25.79±2.06 | 26.48±0.45 |

| | | | | | |
|---|---|---|---|---|---|
| Note: ^{∗} indicates p<0.05 compared with model group. | | | | | |

As shown in Table 4, after the model was established, the food intake of the animals in each model group was significantly decreased compared with that of the normal group, in which the food intake was the least on the 3^{rd} day, then gradually recovered, and basically returned to normal from the 6th day, that meant the food intake was equivalent to that of the normal group. During this period, the food intake of rats in anemoside B4 group was consistently higher than that in the model group, and the difference was statistically significant on days 3 and 4 (p<0.05). However, there was no significant improvement in food intake of dexamethasone group.

### 4.2 Effect of anemoside B4 on the diameter of oral ulcers induced by paclitaxel in rats

The results are shown in Table 5.

**Table 5 Effect of anemoside B4 on the diameter of oral ulcers induced by paclitaxel in rats (x̅ ± s)**

| Groups | | Diameter of oral ulcers (mm) | | | | |
|---|---|---|---|---|---|---|
| | The 2nd day after modeling | The 3rd day after modeling | The 4th day after modeling | The 5th day after modeling | The 6th day after modeling | The 7th day after modeling |
| Normal group | / | / | / | / | / | / |
| Model group | 3.94±0.33 | 4.27±0.53 | 4.02±0.60 | 3.52±0.55 | 3.34±0.46 | 2.80±0.45 |
| Dexamethasone group | 3.93±0.48 | 4.00±0.40 | 3.88±0.58 | 3.50±0.87 | 3.26±0.84 | 2.85±0.81 |
| Anemoside B4 group | 3.94±0.54 | 3.66±0.43* | 3.44±0.76* | 2.73±0.56* | 2.78±0.60* | 2.15±0.44* |

| | | | | | | |
|---|---|---|---|---|---|---|
| Note: ^{∗} indicates p<0.05 compared with model group. | | | | | | |

The experimental data in Table 5 indicated that anemoside B4 suppository could significantly reduce the ulcer surface after administration, having a significant difference (p<0.05) compared with the model group from the 3^{rd} day. There was no significant difference in ulcer diameters between dexamethasone group and model group.

### 5. Conclusion

The above results showed that anemoside B4 had a good therapeutic effect on paclitaxel-induced oral ulcer in rats, could significantly shorten the healing time of the ulcer, and could improve the overall conditions of rats (food intake increased compared with the model group). The above study suggested that anemoside B4 could be used to treat oral ulcers caused by chemotherapeutic drugs in cancer patients, which could reduce patients' pain, improve their quality of life, and enable chemotherapy to be successfully completed.

## Claims

1. Use of anemoside B4 in the preparation of a medicament for the treatment of oral ulcers.

2. Use of anemoside B4 in the preparation of a medicament for the treatment of recurrent oral ulcers.

3. Use of anemoside B4 in the preparation of a medicament for the treatment of oral ulcers caused by chemotherapy.

4. The use according to any one of claims 1 to 3, **characterized in that** anemoside B4 is used as the sole active ingredient in the preparation of the medicaments.

5. The use according to any one of claims 1 to 3, **characterized in that** anemoside B4 is used, together with other active ingredients, in the preparation of the above-mentioned medicaments, in which the other active ingredients are selected from the group consisting of rifampicin, dexamethasone, metronidazole, zinc sulfate, montmorillonite, chlorhexidine, chitosan, sucralfate, polyinosinic-polycytidylic acid (poly I:C), levamisole, vitamin C, B vitamins, granulocyte-macrophage colony stimulating factor (GM-CSF), recombinant human epidermal growth factor (RH EGF), and recombinant human keratinocyte growth factor (rh-KGF).

6. The use according to any one of claims 1 to 5, **characterized in that** the medicament is selected from one or more of oral and non-oral preparations.

7. The use according to claim 6, **characterized in that** the non-oral preparation is selected from the group consisting of injections, rectal preparations, oral mucosa patches, and oral films.

8. The use according to claim 7, **characterized in that** the injection is selected from the group consisting of subcutaneous injections, intramuscular injections, and intravenous injections.

9. The use according to claim 7, **characterized in that** the rectal preparation is selected from the group consisting of rectal suppositories, rectal gels, and rectal perfusion agents.

10. The use according to any one of claims 1 to 9, **characterized in that** the subject using the medicament is a mammal, preferably a human.
